# EUROPÄISCHE PATENTSCHRIFT

(11) **EP 0 590 558 B1**
(45) Veröffentlichungstag und Bekanntmachung des Hinweises auf die Patenterteilung: **06.12.1995**
(21) Anmeldenummer: 93115532.9
(22) Anmeldetag: 27.09.1993
(51) Int. Cl.: A61K 31/415, A61K 31/55

(54) **Arzneimittel enthaltend 1,5,6,7-Tetrahydro-4H-indazol-4-one, neue 1,5,6,7-Tetrahydro-4H-indazol-4-one und verfahren zu ihrer Herstellung**
Pharmaceutical composition containing 1,5,6,7-tetrahydro-4H-indazol-4-ones,new 1,5,6,7-tetrahydro-4H-indazol-4-ones and process for their manufacture
Composition pharmaceutique contenant des tétahydro-1,5,6,7-indazole-4H-ones-4, des nouveaux tétrahydro-1,5,6,7-indazole-4H-ones-4 et procédé pour leur fabricaton

(30) Priorität: 29.09.1992 DE 4232544
(43) Veröffentlichungstag der Anmeldung: 06.04.1994
(73) Patentinhaber: Dr. Karl Thomae GmbH, D-88397 Biberach (DE)
(72) Erfinder: Eberlein, Wolfgang, Dr. Dipl.-Chem., D-88400 Biberach (DE); Engel, Wolfhard, Dr. Dipl.-Chem., D-88400 Biberach (DE); Mihm, Gerhard, Dr. Dipl.-Chem., D-88400 Biberach (DE); Rudolf, Klaus, Dr. Dipl.-Chem., D-88400 Biberach (DE); Engelhardt, Günther, Prof. Dr., D-88400 Biberach (DE)

(56) Entgegenhaltungen:
- EP-A- 0 470 489
- US-A- 3 691 180
- US-A- 4 734 430
- J. HET. CHEM., Bd. 19, 1982, Seiten 1355-1361; PIETRO SCHENONE ET AL.: "Reaction of 2-Dimethylaminomethylene-1,3-diones with dinucleophiles. I. Synthesis of 1, 5-disubstituted 4-acylpyrazoles"

## Beschreibung

Die Erfindung betrifft Arzneimittel enthaltend Pyrazolderivate, insbesondere 1,5,6,7-Tetrahydro-4H-indazol-4-one, neue Verbindungen dieser Struktur, Verfahren zu ihrer Herstellung und die Verwendung dieser Stoffe in Arzneimitteln als Analgetika, Antipyretika und Antiphlogistika.

1,5,6,7-Tetrahydro-4H-indazol-4-one wurden als chemische Verbindungen beschrieben, z.B. in J. Het. Chem. 19, 1355 (1982); J. Org. Chem. 52, 4384 (1987); J. Chem. Research, 1401 (1986); J. Chem. Soc., 803 (1953). In EP 0 470 489 wurden herbizide Verbindungen dieser Strukturklasse beschrieben.

Verschiedene pharmakologische Eigenschaften von 1,5,6,7-Tetrahydro-4H-indazol-4-onen, welche nicht unter die nachstehende allgemeine Formel I fallen, sind z.B. durch folgende Publikationen bekannt:
a.) analgetisch und antiinflammatorisch wirksame Verbindungen durch Il Farmaco, Ed. Sci. 42, 259 (1987); Il Farmaco, Ed. Sci. 43, 763 (1988) und U.S.-Patent Nr. 3 657 438;
b.) broncholytisch und antibiotisch wirksame Verbindungen durch Heterocycles 32, 41 (1991); U.S.-Patent Nr. 4 734 430, U.S.-Patent Nr. 4 734 429;
c.) antiviral wirksame Verbindungen durch U.S.-Patent Nr. 3 691 180.

Es wurde nun gefunden, daß die Verbindungen der Formel 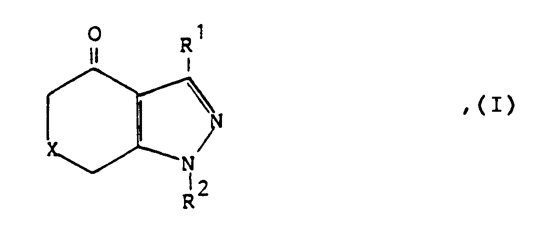
und deren optisch aktive Antipoden, sofern diese ein optisch aktives Kohlenstoffatom enthalten, wertvolle pharmakologische Eigenschaften aufweisen, insbesondere eine analgetische, antipyretische und/oder antiphlogistische Wirkung.

In der obigen Formel I bedeuten
R¹ und R², die gleich oder voneinander verschieden sein können, jeweils ein Wasserstoffatom, eine gegebenenfalls durch eine Phenyl-, Fluorphenyl-, Chlorphenyl- oder Bromphenylgruppe substituierte geradkettige oder verzweigte Alkylgruppe mit 1 bis 6 Kohlenstoffatomen, die Phenylgruppe, welche gegebenenfalls noch durch ein Halogenatom substituiert sein kann, mit der Maßgabe, daß R² keine Halogenphenylgruppe darstellt, wenn R¹ ein Wasserstoffatom darstellt,
X eine Einfachbindung oder eine Methylengruppe, die gegebenenfalls durch eine oder zwei Alkylgruppen mit 1 bis 3 Kohlenstoffatomen substituiert sein kann, oder eine Ethylengruppe.

Hierbei sind diejenigen Verbindungen der Formel I neu, in denen
R¹ ein Wasserstoffatom, R² eine geradkettige oder verzweigte Alkylgruppe mit 2 bis 6 Kohlenstoffatomen und X eine Methylengruppe, die durch eine oder zwei Methylgruppen substituiert sein kann, bedeuten,
sowie diejenigen Verbindungen, in denen
R¹ einen Phenylrest, der gegebenenfalls noch durch ein Halogenatom substituiert sein kann, R² eine geradkettige oder verzweigte Alkylgruppe mit 1 bis 6 Kohlenstoffatomen, die noch durch einen Phenyl- oder Halogenphenylrest substituiert sein kann, und X eine Methylgruppe, die durch eine oder zwei Methylgruppen substituiert sein kann, bedeuten, mit der Maßgabe, daß X eine durch eine oder zwei Methylgruppen substituierte Methylengruppe darstellt, falls R¹ einen Phenylrest und R² eine Methylgruppe darstellt,
sowie diejenigen Verbindungen, in denen
R¹ und R², die gleich oder voneinander verschieden sein können, jeweils ein Wasserstoffatom, eine gegebenenfalls durch eine Phenyl-, Fluorphenyl-, Chlorphenyl- oder Bromphenylgruppe substituierte geradkettige oder verzweigte Alkylgruppe mit 1 bis 6 Kohlenstoffatomen, die Phenylgruppe, welche gegebenenfalls noch durch ein Halogenatom substituiert sein kann, mit der Maßgabe, daß R² keine Halogenphenylgruppe darstellt, wenn R¹ ein Wasserstoffatom darstellt, und X eine Einfachbindung oder die Ethylengruppe, mit der Maßgabe, daß X keine Einfachbindung darstellt, wenn R¹ ein Wasserstoffatom und R² eine Phenylgruppe darstellt, bedeuten.

Gegenstand der vorliegenden Erfindung sind somit die neuen Arzneimittel, enthaltend eine Verbindung der Formel I, welche zur Bekämpfung von Schmerzzuständen, von Fieber und Entzündungen sowie zur Bekämpfung der Symptomatik von Erkältungskrankheiten geeignet sind, die neuen Verbindungen der vorstehenden Formel I und Verfahren zu ihrer Herstellung.

Ein weiterer Gegenstand der Erfindung ist die Verwendung der Verbindungen der allgemeinen Formel I, in der R¹, R² und X wie vorstehend erwähnt definiert sind oder in denen R¹ ein Wasserstoffatom, R² eine durch ein Halogenatom substituierte Phenylgruppe und X eine Einfachbindung oder eine Methylengruppe, die gegebenenfalls durch eine oder zwei Alkylgruppen mit 1 bis 3 Kohlenstoffatomen substituiert sein kann, oder eine Ethylengruppe bedeuten, zur Herstellung eines Arzneimittels, das zur Bekämpfung von Fieber und Entzündungen sowie zur Bekämpfung der Symptomatik von Erkältungskrankheiten geeignet ist.

Besonderer Wirkungen wegen bevorzugte Verbindungen der Formel I sind diejenigen, für die
- R¹: ein Wasserstoffatom oder die Methylgruppe,
- R²: die Methyl-, Ethyl-, Propyl-, Isopropyl-, Benzyl- oder die Phenylgruppe und
- X: die Methylen- oder die mit 2 Methylgruppen substituierte Methylengruppe bedeuten.

Ganz besonders bevorzugt ist die Verbindung der Formel I, in der R¹ ein Wasserstoffatom, R² die Methylgruppe und X die Methylengruppe darstellen. Diese Verbindung ist als Ausgangsverbindung zur Herstellung von analgetisch und antiinflammatorisch wirksamen Verbindungen in Il Farmaco Ed. Sci. 42, 259 (1987) und Il Farmaco Ed. Sci. 47, 567 (1992) vorbeschrieben, über etwaige pharmakologische Eigenschaften dieser Ausgangsverbindung selbst ist aber dort nichts ausgesagt worden. Es ist deshalb sehr überraschend, daß diese Verbindung selbst sehr gute analgetische, antipyretische und antiphlogistische Eigenschaften besitzt, was bisher nicht erkannt wurde.

Im U.S.-Patent Nr. 3 657 438 dient die Verbindung der Formel I, in der R¹ die Methylgruppe, R² die Benzylgruppe und X die Methylengruppe darstellt, als Ausgangsverbindung zur Herstellung von antiinflammatorisch wirksamen Verbindungen; über eigenständige pharmakologische Eigenschaften der Ausgangsverbindung wurde nichts angegeben. Es wurde nun überraschenderweise auch für diese Verbindung gefunden, daß sie die oben angeführten wertvollen Eigenschaften besitzt.

Die Herstellung von Verbindungen der Formel I, in der R¹ ein Wasserstoffatom, R² die Methyl- oder Phenylgruppe und X die Methylen- oder Dimethylmethylengruppe darstellen, ist in J. Het. Chem. 19, 1355 (1982) beschrieben.

Die Herstellung der Verbindung der Formel I, in der R¹ ein Wasserstoffatom, R² die Benzylgruppe und X die Methylengruppe bedeutet, ist durch Heterocycles 32, 41 (1991) bekannt. Die Herstellung der Verbindung der Formel I, in der R¹ die Methylgruppe, R² die Phenylgruppe und X die Methylengruppe darstellen, geht aus J. Chem. Soc. 803 (1953) hervor.

Die vorstehend erwähnten neuen Verbindungen erhält man erfindungsgemäß nach folgenden Verfahren:
a.) Herstellung von Verbindungen der Formel I, in der R¹ die eingangs erwähnten Bedeutungen mit Ausnahme der eines Wasserstoffatoms besitzt, durch Umsetzung einer Verbindung der Formel II 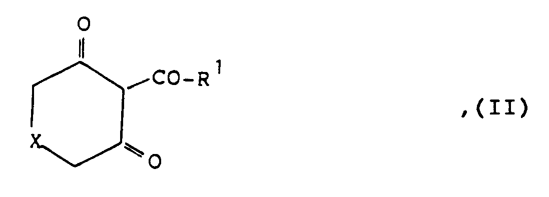
in der X wie oben definiert ist und R¹ die obengenannten Bedeutungen mit Ausnahme der eines Wasserstoffatoms besitzt, mit einem Hydrazin der Formel III

H₂N - NH - R² ,(III)

in der R² ebenfalls wie oben definiert ist.

Die Umsetzung wird vorzugsweise in einem Lösungsmittel wie Methanol, Ethanol, Isopropanol, Tetrahydrofuran, Dioxan, Methylenchlorid oder Benzol, bei Temperaturen zwischen 0°C und der Rückflußtemperatur des Reaktionsgemisches durchgeführt. Die Isolierung der Endprodukte erfolgt in an sich bekannter Weise durch Entfernung des Lösungsmittels.
b.) Herstellung von Verbindungen der Formel I, in der R¹ ein Wasserstoffatom bedeutet, durch Umsetzung einer Verbindung der Formel IV

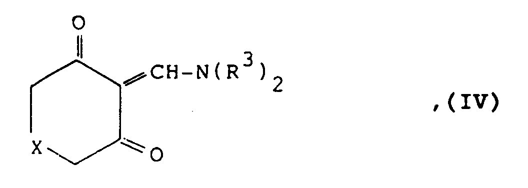

in der X wie oben definiert ist und R³ eine Alkylgruppe, vorzugsweise die Methylgruppe, bedeutet, mit einem Hydrazin der vorstehend genannten Formel III.

Das Hydrazin der Formel III und die Verbindung der Formel IV werden in Methanol, Ethanol, Isopropanol, Tetrahydrofuran, Dioxan, Methylenchlorid gelöst und die Lösungen bei Temperaturen um 0°C vereint. Nach mehrstündigem Rühren bei Temperaturen zwischen 0°C und der Rückflußtemperatur des Reaktionsgemisches wird das Lösungsmittel, z.B. durch Abdestillieren desselben, entfernt.

Die Ausgangssubstanzen gemäß Formel II lassen sich in Anlehnung an die in J. Chem. Soc. 803 (1953) und Synthesis 925 (1978) beschriebenen Verfahrensweisen herstellen. Zur Herstellung der Ausgangssubstanzen der Formel IV wird auf J. Het. Chem. 19, 1355 (1982) verwiesen.

Wie bereits eingangs erwähnt, weisen die Verbindungen der allgemeinen Formel I wertvolle pharmakologische Eigenschaften auf, insbesondere antipyretische, analgetische und antiphlogistische Eigenschaften.

### Insbesondere wurde die Verbindung

### 1-Methyl-1,5,6,7-tetrahydro-4H-indazol-4-on = Substanz A

wie folgt geprüft:
1. Die Prüfung der Wirkung gegen den Entzündungsschmerz der Ratte erfolgte mit der Methode nach RANDALL u. SELITTO (Arch. int. Pharmacodyn. 111, 409 (1957)). Die Prüfsubstanzen wurden männlichen Ratten mit einem Gewicht zwischen 100 und 130 g als Verreibung in 1%iger Methylcellulose (1,0 ml/100 g Tier) 90 Minuten nach subcutaner Gabe der Hefe per Schlundsonde verabfolgt. Aus der 45 bzw. 90 bzw. 180 Minuten nach Gabe der verschiedenen Dosen gemessenen Schmerzschwelle wurde nach linearer Regressionsanalyse eine ED₅₀ als jene Dosis ermittelt, die eine Anhebung der Schmerzschwelle um 50 % bewirkte.

Die nachfolgende Tabelle enthält die gefundenen Werte:

| Substanz | ED₅₀ mg/kg | |
|---|---|---|
| | nach 45 Min. | nach 90 Min. |
| A | 3,1 | 2,1 |

2. Die Prüfung der Wirkung gegen den Wärmeschmerz der Maus erfolgte mit der Methode nach CHEN und BECKMAN (Science 113, 631 (1951)). Männliche Mäuse mit einem durchschnittlichen Gewicht von 20 g erhielten die Prüfsubstanzen als Verreibung in 1%iger Methylcellulose (0,1 ml/10 g Tier) per Schlundsonde. Aus der nach den verschiedenen Dosen beobachteten Verlängerung der individuellen Reaktionszeit wurde nach linearer Regressionsanalyse eine ED₁₀₀ als jene Dosis berechnet, die zu einer Verdoppelung der Reaktionszeit führte.

Die nachfolgende Tabelle enthält die gefundenen Werte:

| Substanz | ED₁₀₀ mg/kg |
|---|---|
| A | 159 |

3. Die Prüfung der Wirkung gegen den mechanisch ausgelösten Schmerz erfolgte mit der Schwanzklemmenmethode nach HAFFNER (Dtsch. med. Wschr. 54, 731 (1929)). Männliche Mäuse mit einem Gewicht zwischen 19 und 24 g erhielten die Prüfsubstanzen als Verreibung in 1%iger Methylzellulose (0,1 ml/10 g Tier) per Schlundsonde. In Abständen von 30 Minuten wurde nach der Behandlung festgestellt, wieviel Mäuse nicht mehr auf das Anlegen der Klemme reagierten.

Aus dem Prozentsatz der Tiere, die nach den verschiedenen Dosen keine Schmerzreaktion zeigten, wurde mittels Probit-Analyse eine ED₅₀ berechnet.

Die nachfolgende Tabelle enthält die gefundenen Werte:

| Substanz | ED₅₀ mg/kg |
|---|---|
| A | 45,2 |

4. Die Prüfung der Wirkung auf die Körpertemperatur erfolgte an normothermen Ratten mit einem Gewicht zwischen 120 und 140 g. Die Prüfsubstanzen wurden als Verreibung in 1%iger Methylcellulose (1,0 ml/100 g Tier) per Schlundsonde verabfolgt. Aus der nach den verschiedenen Dosen beobachteten Senkung der Rektaltemperatur wurde nach linearer Regressionsanalyse eine ED_{-1,5°C} als jene Dosis berechnet, die eine Senkung der Körpertemperatur um 1,5°C bewirkte.

Die nachfolgende Tabelle enthält die gefundenen Werte:

| Substanz | ED_{-1,5°C} mg/kg |
|---|---|
| A | 7,1 |

5. Die Bestimmung der akuten Toxizität erfolgte an Mäusen oder Ratten beider Geschlechter mit einem mittleren Gewicht von 20 g. Die Prüfsubstanzen wurden als Verreibung in 1%iger Methylcellulose (0,2 ml/10 g Tier) per Schlundsonde verabfolgt. Die Berechnung der LD₅₀ erfolgte (soweit möglich) nach LITCHFIELD u. WILCOXON (J. Pharmacol. exp. Therap. 96, 99 (1949) aus dem Prozentsatz der Tiere, die innerhalb von 14 Tagen nach den verschiedenen Dosen verstarben.

Die nachfolgende Tabelle enthält die gefundenen Werte:

| Substanz | LD₅₀ mg/kg | |
|---|---|---|
| | Maus | Ratte |
| A | 1670 | 1090 |

Aufgrund ihrer pharmakologischen Eigenschaften stellen die Verbindungen der allgemeinen Formel I Analgetica/Antipyretica vom Typ des Aminophenazons dar. Sie eignen sich daher zur Bekämpfung von Schmerzzuständen wie Kopf-, Zahn-, Regel-, Nervenschmerzen, Migräne, postoperativen und posttraumatischen Schmerzen, aber auch zur Bekämpfung von Fieber und Entzündungen bzw. der Symptomatik von Erkältungskrankheiten.

Hierzu lassen sich die Verbindungen der allgemeinen Formel I, gegebenenfalls in Kombination mit anderen Wirkstoffen, zusammen mit einem oder mehreren inerten Trägerstoffen und/oder Verdünnungsmitteln, z.B. mit Wasser, Maisstärke, Kartoffelstärke, Milchzucker, Rohrzucker, mikrokristalliner Cellulose, Magnesiumstearat, Polyvinylpyrrolidon, Hartfett, Carboxymethylcellulose oder deren geeigneten Gemischen, in übliche galenische Zubereitungen wie Tabletten, Dragées, Kapseln, Pulver, Zäpfchen, Suspensionen und Lösungen einarbeiten. Hierbei beträgt die Einzeldosis am Erwachsenen 25-1200 mg, zweckmäßigerweise 50-600 mg, vorzugsweise jedoch 100 bis 300 mg.

Die nachfolgenden Beispiele sollen die Erfindung näher erläutern:

### Beispiel 1

### 1-Methyl-1,5,6,7-tetrahydro-4H-indazol-4-on

Eine Lösung von 46,1 g (1,0 Mol) Methylhydrazin in 400ml Methanol wird bei Raumtemperatur in eine Lösung von 167,2 g (1,0 Mol) 2-(Dimethylaminomethylen)-cyclohexan-1,3-dion in 2000 ml Methanol langsam eingetropft. Die Reaktionsmischung wird anschließend während 1,5 Stunden unter Rückfluß gerührt. Man engt im Vakuum zur Trockene ein und reinigt das erhaltene Rohprodukt durch Chromatographie an Kieselgel (Fa. Baker, 30-60 »m) mit einem Gemisch aus Essigester/Cyclohexan = 9:1.

Durch Umkristallisieren aus Diisopropylether erhält man die gewünschte Verbindung in einer Ausbeute von 89,3 % der Theorie. Farblose Kristalle vom Fp. 96-97°C.

### Beispiel 2

### 1-Ethyl-1,5,6,7-tetrahydro-4H-indazol-4-on

Hergestellt analog Beispiel 1 ausgehend von Ethylhydrazin und 2-(Dimethylaminomethylen)-cyclohexan-1,3-dion.

| | |
|---|---|
| Schmelzpunkt: | 60-61°C |
| Ausbeute: | 88 % der Theorie |

### Beispiel 3

### 1-Propyl-1,5,6,7-tetrahydro-4H-indazol-4-on

Hergestellt analog Beispiel 1 ausgehend von Propylhydrazin und 2-(Dimethylaminomethylen)-cyclohexan-1,3-dion.

| | |
|---|---|
| Schmelzpunkt: | 47-48°C |
| Ausbeute: | 70 % der Theorie |

### Beispiel 4

### 1-(1-Methylethyl)-1,5,6,7-tetrahydro-4H-indazol-4-on

Hergestellt analog Beispiel 1 ausgehend von Isopropylhydrazin und 2-(Dimethylaminomethylen)-cyclohexan-1,3-dion.

| | |
|---|---|
| Schmelzpunkt: | 84-85°C |
| Ausbeute: | 58 % der Theorie |

### Beispiel 5

### 1-Benzyl-1,5,6,7-tetrahydro-4H-indazol-4-on

Hergestellt analog Beispiel 1 ausgehend von Benzylhydrazin und 2-(Dimethylaminomethylen)-cyclohexan-1,3-dion.

| | |
|---|---|
| Schmelzpunkt: | 60°C |
| Ausbeute: | 72 % der Theorie |

### Beispiel 6

### 1-Phenyl-1,5,6,7-tetrahydro-4H-indazol-4-on

Hergestellt analog Beispiel 1 ausgehend von Phenylhydrazin und 2-(Dimethylaminomethylen)-cyclohexan-1,3-dion.

| | |
|---|---|
| Schmelzpunkt: | 140°C |
| Ausbeute: | 49 % der Theorie |

### Beispiel 7

### 1,6,6-Trimethyl-1,5,6,7-tetrahydro-4H-indazol-4-on

Hergestellt analog Beispiel 1 ausgehend von Methylhydrazin und 2-(Dimethylaminomethylen)-5,5-dimethyl-cyclohexan-1,3-dion.

| | |
|---|---|
| Schmelzpunkt: | 74-75°C |
| Ausbeute: | 32 % der Theorie |

### Beispiel 8

### 1-Ethyl-3-methyl-1,5,6,7-tetrahydro-4H-indazol-4-on

Hergestellt analog Beispiel 1 ausgehend von 2-Acetyl-cyclohexan-1,3-dion und Ethylhydrazin.

| | |
|---|---|
| Schmelzpunkt: | 66-67°C |
| Ausbeute: | 43 % der Theorie |

### Pharmazeutische Beispiele

### Beispiel I

### Tabletten mit 125 mg 1-Methyl-1,5,6,7-tetrahydro-4H-indazol-4-on

### Zusammensetzung:

| | |
|---|---|
| Wirkstoff | 125,0 mg |
| mikrokristalline Cellulose | 63,5 mg |
| Milchzucker, direkttablettierbar | 110,0 mg |
| Magnesiumstearat | 1,5 mg |
| | 3̅0̅0̅,̅0̅ m̅g̅ |

### Herstellung:

Die Hilfsstoffe werden mit dem Wirkstoff gründlich gemischt und zu Tabletten verpreßt. Man erhält runde, biplane Tabletten mit beidseitiger Facette und einseitiger Teilkerbe.

| | |
|---|---|
| Tabletten-Gewicht: | ca. 300 mg |
| Tabletten-Durchmesser: | 10 mm |

### Beispiel II

### Dragees mit 125 mg 1-Methyl-1,5,6,7-tetrahydro-4H-indazol-4-on

### Zusammensetzung:

| | |
|---|---|
| Wirkstoff | 125,0 mg |
| mikrokristalline Cellulose | 63,5 mg |
| Milchzucker, direkttablettierbar | 110,0 mg |
| Magnesiumstearat | 1,5 mg |
| | 3̅0̅0̅,̅0̅ m̅g̅ |

### Herstellung:

Die Hilfsstoffe werden mit dem Wirkstoff gründlich gemischt und zu Dragees verpreßt. Die erhaltenen Dragees werden mit üblicher Zuckerdragiersuspension und anschließend mit reinem Zuckersirup auf 390 mg/Dragee in einem Dragierkessel dragiert.

| | |
|---|---|
| Drageekern-Gewicht: | ca. 300 mg |
| Drageekern-Durchmesser: | 10 mm |
| Aussehen: | rund, bikonvex |

### Beispiel III

### Granulat mit 1-Methyl-1,5,6,7-tetrahydro-4H-indazol-4-on

### Zusammensetzung:

| | |
|---|---|
| (01) Wirkstoff | 12,5 % |
| (02) Sorbit | 86,0 % |
| (03) Siliciumdioxid | 1,3 % |
| (04) Magnesiumstearat | 0,2 % |
| | 1̅0̅0̅,̅0̅ %̅ |

### Herstellung:

Die Komponenten (01), (02) und (03) werden gemischt und mit Äthanol feucht granuliert. Nach Trocknung und Sieben (Maschenweite 1,0 mm) wird das Granulat mit (04) gemischt und in Sachets abgefüllt. Ein Sachet mit 1 g Granulat enthält 125 mg Wirkstoff.

### Beispiel IV

### "Fine Granules" mit 1-Methyl-1,5,6,7-tetrahydro-4H-indazol-4-on

### Zusammensetzung:

| | |
|---|---|
| (01) Wirkstoff | 12,5 % |
| (02) Polyvinylpyrrolidon | 2,8 % |
| (03) Lactose | 83,0 % |
| (04) Siliciumdioxid | 1,2 % |
| (05) Magnesiumstearat | 0,5 % |
| | 1̅0̅0̅,̅0̅ %̅ |

### Herstellung:

In einem rotierenden Dragierkessel wird (03) mit einer Teilchengröße von 0,2-0,45 mm vorgelegt.
In Isopropanol wird (02) gelöst und anschließend (01) und (04) suspendiert. Die Suspension wird unter Zuführung von Trockenluft vorsichtig auf die im Kessel fließenden Lactose-Kristalle aufgesprüht. Nach Trocknung mischt man mit (05) und füllt eine 125 mg Wirkstoff entsprechende Menge Granules in Sachets ab.

### Beispiel V

### Injektionslösung mit 125 mg 1-Methyl-1,5,6,7-tetrahydro-4H-indazol-4-on

### Zusammensetzung:

| | |
|---|---|
| Wirkstoff | 125,0 mg |
| Wasser für Injektionszwecke ad | 2 ml |

### Herstellung:

Der Wirkstoff wird bei Raumtemperatur in Wasser für Injektionszwecke gelöst. Die Lösung wird sterilfiltriert, in gereinigte Ampullen abgefüllt und bei 121°C 20 Minuten lang autoklaviert.

### Beispiel VI

### Suppositorien mit 200 mg 1-Methyl-1,5,6,7-tetrahydro-4H-indazol-4-on

### Zusammensetzung:

| | |
|---|---|
| Wirkstoff | 200,0 mg |
| Hartfett (z.B. Witepsol® H 19 und Witepsol® W 45) | 1.500,0 mg |

### Herstellung:

Das Hartfett wird geschmolzen. Bei 38°C wird die gemahlene Wirksubstanz in der Schmelze homogen dispergiert. Es wird auf 35°C abgekühlt und in schwach vorgekühlte Suppositorienformen ausgegossen.

### Beispiel VII

### Saft mit 125 mg 1-Methyl-1,5,6,7-tetrahydro-4H-indazol-4-on

### Zusammensetzung:

| | |
|---|---|
| Wirkstoff | 2,50 g |
| Carboxymethylcellulose | 0,10 g |
| p-Hydroxybenzoesäuremethylester | 0,05 g |
| p-Hydroxybenzoesäurepropylester | 0,03 g |
| Saccharose | 10,00 g |
| Glycerin | 5,00 g |
| Sorbitlösung 70 % | 20,00 g |
| Aroma | 0,30 g |
| Wasser dest. ad | 100,00 ml |

### Herstellung:

Destilliertes Wasser wird auf 70°C erhitzt. Hierin wird unter Rühren p-Hydroxybenzoesäuremethylester und -propylester sowie Glycerin und Carboxymethylcellulose gelöst. Es wird auf Raumtemperatur abgekühlt und unter Rühren der Wirkstoff zugegeben und gelöst. Nach Zugabe und Lösen der Saccharose, der Sorbitlösung und des Aromas wird der Saft zur Entlüftung unter Rühren evakuiert.

## Patentansprüche

1. Arzneimittel, enthaltend eine Verbindung der Formel 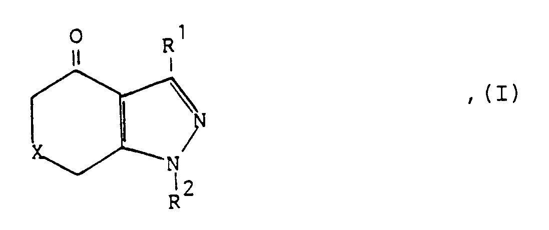 in der
R¹ und R², die gleich oder voneinander verschieden sein können, jeweils ein Wasserstoffatom, eine gegebenfalls durch eine Phenyl-, Fluorphenyl-, Chlorphenyl- oder Bromphenylgruppe substituierte geradkettige oder verzweigte Alkylgruppe mit 1 bis 6 Kohlenstoffatomen, die Phenylgruppe, welche gegebenenfalls noch durch ein Halogenatom substituiert sein kann, mit der Maßgabe, daß R² keine Halogenphenylgruppe darstellt, wenn R¹ ein Wasserstoffatom darstellt,
X eine Einfachbindung oder eine Methylengruppe, die gegebenenfalls durch eine oder zwei Alkylgruppen mit 1 bis 3 Kohlenstoffatomen substituiert sein kann, oder eine Ethylengruppe
bedeuten, neben einem oder mehreren inerten Trägerstoffen und/oder Verdünnungsmitteln.

2. Arzneimittel gemäß Anspruch 1, enthaltend eine Verbindung der Formel I, in der
R¹ ein Wasserstoffatom oder die Methylgruppe,
R² die Methyl-, Ethyl-, Propyl-, Isopropyl-, Benzyl- oder die Phenylgruppe und
X die Methylen- oder die mit 2 Methylgruppen substituierte Methylengruppe bedeuten.

3. Arzneimittel gemäß Anspruch 1 und 2, enthaltend eine Verbindung der Formel I, in der
R¹ ein Wasserstoffatom, R² die Methylgruppe und
X die Methylengruppe darstellen.

4. Arzneimittel nach mindestens einem der Ansprüche 1 bis 3, dadurch gekennzeichnet, daß diese zur Bekämpfung von Schmerzzuständen, zur Bekämpfung von Fieber und Entzündungen sowie zur Bekämpfung der Symptomatik von Erkältungskrankheiten geeignet sind.

5. Verwendung der Verbindungen nach mindestens einem der Ansprüche 1 bis 3 oder einer Verbindung der allgemeinen Formel 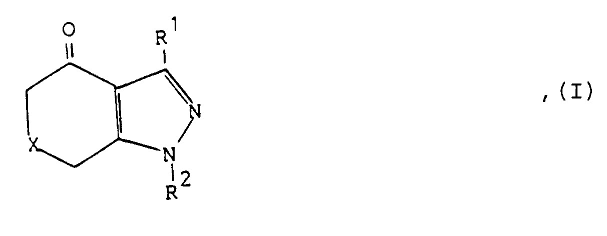 in der
R¹ ein Wasserstoffatom,
R² eine durch ein Halogenatom substituierte Phenylgrupe und
X eine Einfachbindung oder eine Methylengruppe, die gegebenenfalls durch eine oder zwei Alkylgruppen mit 1 bis 3 Kohlenstoffatomen substituiert sein kann, oder eine Ethylengruppe
bedeuten, zur Herstellung eines Arzneimittels, das zur Bekämpfung von Schmerzzuständen, zur Bekämpfung von Fieber und Entzündungen sowie zur Bekämpfung der Symptomatik von Erkältungskrankheiten geeignet ist.

6. Verfahren zur Herstellung eines Arzneimittels nach mindestens einem der Ansprüche 1 bis 3, dadurch gekennzeichnet, daß auf nichtchemischem Wege eine Verbindung nach mindestens einem der Ansprüche 1 bis 3 in einen oder mehrere inerte Trägerstoffe und/oder Verdünnungsmittel eingearbeitet wird.

7. 1,5,6,7-Tetrahydro-4H-indazol-4-one der Formel 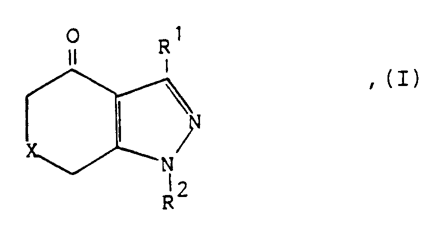 in der
R¹ ein Wasserstoffatom und R² eine geradkettige oder verzweigte Alkylgruppe mit 2 bis 6 Kohlenstoffatomen und X eine Methylengruppe, die durch eine oder zwei Methylgruppen substituiert sein kann, bedeuten.

8. 1,5,6,7-Tetrahydro-4H-indazol-4-one der Formel 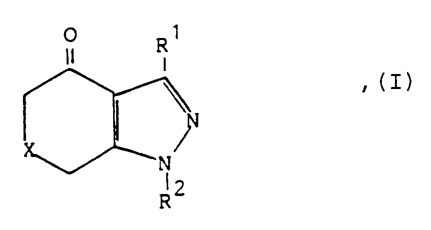 in der
R¹ einen Phenylrest, der gegebenenfalls noch durch ein Halogenatom substituiert sein kann und R² eine geradkettige oder verzweigte Alkylgruppe mit 1 bis 6 Kohlenstoffatomen, die noch durch einen Phenyl- oder Halogenphenylrest substituiert sein kann, und
X eine Methylengruppe, die durch eine oder zwei Methylgruppen substituiert sein kann, bedeuten, mit der Maßgabe, daß X eine durch eine oder zwei Methylgruppen substituierte Methylengruppe darstellt, falls R¹ einen Phenylrest und R² eine Methylgruppe darstellt.

9. Pyrazolderivate der Formel 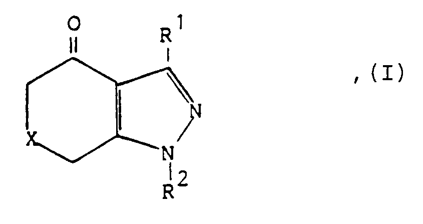 in der
R¹ und R² wie in Anspruch 1 definiert sind und X eine Einfachbindung oder die Ethylengruppe darstellt, mit der Maßgabe, daß X keine Einfachbindung darstellt, wenn R¹ ein Wasserstoffatom und R² eine Phenylgruppe darstellt.

10. Verfahren zur Herstellung der Verbindungen der Formel I gemäß den Ansprüchen 7, 8 und 9, dadurch gekennzeichnet, daß
a.) zur Herstellung von Verbindungen der Formel I, in der R¹ die in den Ansprüchen 7 bis 9 erwähnten Bedeutungen mit Ausnahme der eines Wasserstoffatoms besitzt, eine Verbindung der Formel II 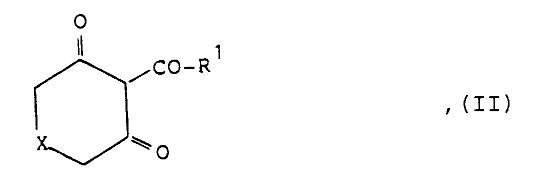 in der X wie in den Ansprüchen 7 bis 9 definiert ist und R¹ die in den Ansprüchen 7 bis 9 genannten Bedeutungen mit Ausnahme der eines Wasserstoffatoms besitzt, mit einem Hydrazin der Formel III
H₂N - NH - R² ,(III)
in der R² wie in den Ansprüchen 7 bis 9 definiert ist, umgesetzt oder
b.) zur Herstellung von Verbindungen der Formel I, in der R¹ ein Wasserstoffatom bedeutet, eine Verbindung der Formel IV 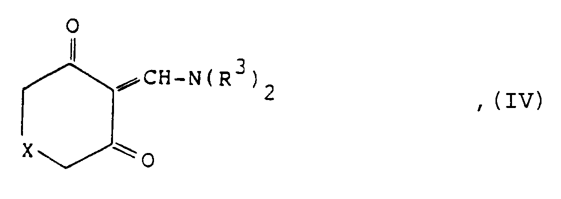 in der X wie in den Ansprüchen 7 bis 9 definiert ist und R³ eine Alkylgruppe bedeutet, mit einem Hydrazin der Formel III umgesetzt wird.

## Claims

1. A Pharmaceutical composition comprising a compound of the formula I 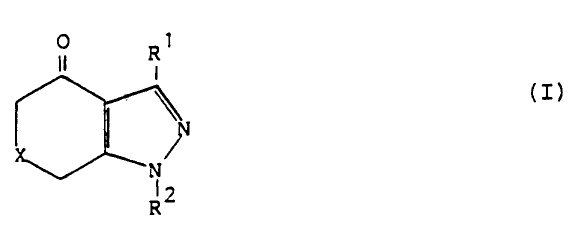 wherein
R¹ and R², which may be identical or different, each represents a hydrogen atom, a straight-chained or branched C₁₋₆-alkyl group optionally substituted by a phenyl, fluorophenyl, chlorophenyl or bromophenyl group, or a phenyl group which may optionally be substituted by a halogen atom, subject to the proviso that R² does not represent a halogenphenyl group if R¹ represents a hydrogen atom, and
X represents a single bond, a methylene group which may optionally be substituted by one or two C₁₋₃-alkyl groups, or an ethylene group,
together with one or more physiologically acceptable carriers and/or excipients.

2. A composition as claimed in claim 1, containing a compound of formula I wherein
R¹ denotes a hydrogen atom or a methyl group,
R² denotes a methyl, ethyl, propyl, isopropyl, benzyl or phenyl group and
X denotes a methylene group or a methylene group substituted by 2 methyl groups.

3. A composition as claimed in either of claims 1 and 2, containing a compound of formula I wherein
R¹ denotes a hydrogen atom, R² a methyl group and X a methylene group,

4. A composition as claimed in any one or more of claims 1 to 3, characterised in that it is suitable for combating painful conditions, for combating fever and inflammations as well as for combating the symptoms of catarrhal diseases.

5. Use of the compounds according to at least one of claims 1 to 3, or a compound of the general formula 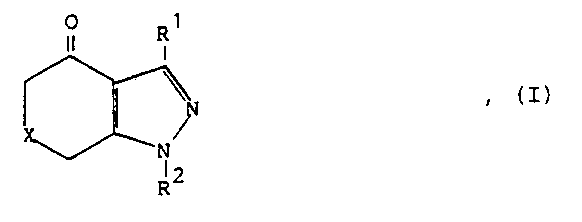 in which R¹ represents a hydrogen atom,
R² represents a phenyl group substituted by a halogen atom, and
X represents a single bond or a methylene group optionally substituted by one or two C₁₋₃-alkyl groups, or an ethylene group, for the manufacture of a pharmaceutical composition for combating painful conditions, for combating fever and inflammations as well as for combating the symptoms of catarrhal diseases.

6. Process for the manufacture of a pharmaceutical compositions as claimed in any one or more of claims 1 to 3, wherein in a non-chemical manner a compound, according to any one or more of claims 1 to 3, is incorporated with one or more inert carriers and/or excipients.

7. A 1,5,6,7-tetrahydro-4H-indazol-4-one compound of the formula 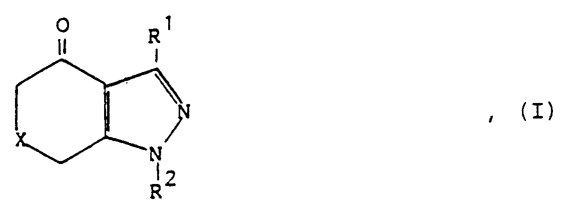 in which R¹ represents a hydrogen atom and R² represents a straight chain or branched C₂₋₆-alkyl group and X represents a methylene group which may be substituted by one or two methyl groups.

8. A 1,5,6,7-tetrahydro-4H-indazol-4-one compound of the formula 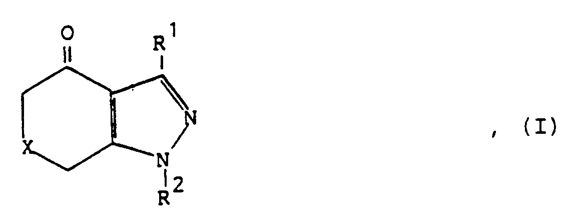 in which R¹ represents a phenyl group which may optionally be substituted by a halogen atom and wherein R² represents a straight chain or branched C₁₋₆-alkyl group which may be substituted by one phenyl or halogenphenyl group, and
X represents a methylene group, which may be substituted by one or two methyl groups, subject to the proviso that, in the event that R¹ represents a phenyl group and R² represents a methyl group, X represents a methylene group substituted by one or two methyl groups.

9. Pyrazole derivatives of the formula 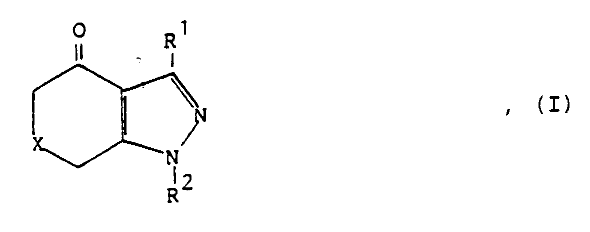 in which R¹ and R² are as defined in claim 1 and X represents a single bond or the ethylene group, subject to the proviso that X does not represent a single bond if R¹ represents a hydrogen atom and R² represents a phenyl group.

10. Process for the preparation of the compounds of formula I according to claims 7, 8 and 9 wherein
a) for the manufacture of compounds of the formula I wherein R¹ has the meanings as set out in claims 7 to 9, except for that of a hydrogen atom, a compound of the formula II, 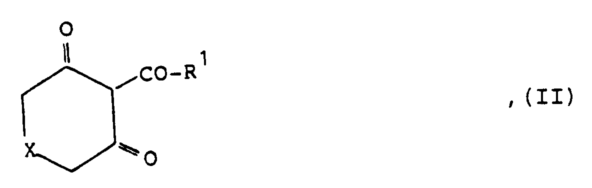 wherein X is as defined in claims 7 to 9 and R¹ has the meanings as set out in claims 7 to 9, except for that of a hydrogen atom, is reacted with a hydrazine of the formula III
H₂N-NH-R² ,(III)
in which R² has the meaning as defined in claims 7 to 9 or
b) for the manufacture of compounds of formula I in which R¹ denotes a hydrogen atom a compound of the formula IV 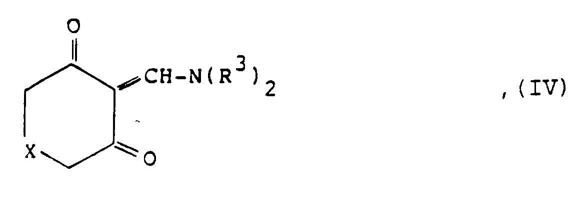 in which X is as defined in claims 7 to 9 and R³ represents an alkyl group is reacted with a hydrazine of formula III.

## Revendications

1. Médicament, comprenant un composé de la formule 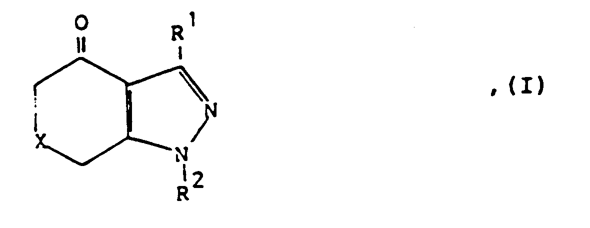 dans laquelle
R¹ et R², qui peuvent être identiques ou différents l'un de l'autre, signifient chaque fois un atome d'hydrogène, un groupe alkyle linéaire ou ramifié, éventuellement substitué par un groupe phényle, fluorophényle, chlorophényle ou bromophényle avec 1 à 6 atomes de carbone, le groupe phényle pouvant éventuellement être encore substitué par un atome halogène, dans la mesure où R² ne représente pas un groupe halogénophényle, lorsque R¹ représente un atome d'hydrogène,
X représente une liaison simple ou un groupe méthyle qui peut éventuellement être substitué par un ou deux groupes alkyle avec 1 à 3 atomes de carbone, ou un groupe éthylène, en plus d'un ou de plusieurs matériaux porteurs inertes et/ou moyens de dilution.

2. Médicament selon la revendication 1, contenant un composé de la formule I, dans laquelle
R¹ signifie un atome hydrogène ou le groupe méthyle,
R² le groupe méthyle, éthyle, propyle, isopropyle, benzyle ou phényle et
X le groupe méthylène ou le groupe méthylène substitué par 2 groupes méthyle.

3. Médicament selon la revendication 1 ou 2, contenant un composé de la formule I, dans laquelle
R¹ représente un atome d'hydrogène, R² le groupe méthyle et
X le groupe méthylène.

4. Médicaments selon au moins l'une des revendications 1 à 3, caractérisés en ce que ceux-ci sont appropriés à la lutte contre des états douloureux, à la lutte contre la fièvre et les inflammations ainsi qu'à la lutte contre les symptômes d'affections dues à un refroidissement.

5. Utilisation des composés selon au moins l'une des revendications 1 à 3 ou d'un composé de la formule générale 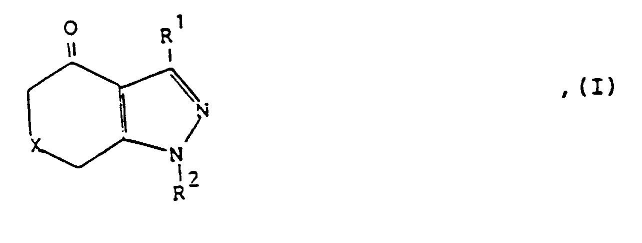 dans laquelle
R¹ signifie un atome hydrogène,
R² un groupe phényle substitué par un atome halogène et
X une liaison simple ou un groupe méthylène qui peut être éventuellement substitué par un ou deux groupes alkyle avec 1 à 3 atomes de carbone, ou un groupe éthylène, pour la préparation d'un médicament qui est approprié à la lutte contre les états de douleur, à la lutte contre la fièvre et les inflammations ainsi qu'à la lutte contre les symptômes des affections dues à un refroidissement.

6. Procédé pour la préparation d'un médicament selon au moins l'une des revendications 1 à 3, caractérisé en ce que l'on introduit par des voies non chimiques un compose selon au moins l'une des revendications 1 à 3 dans un ou plusieurs excipients inertes et/ou diluants.

7. 1,5,6,7-tétrahydro-4H-indazol-4-one de la formule 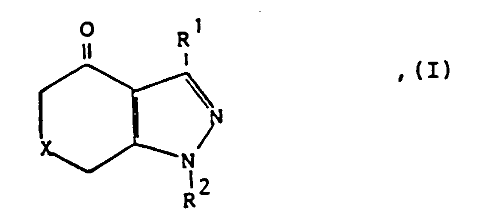 dans laquelle
R¹ représente un atome d'hydrogène et R² un groupe alkyle linéaire ou ramifié avec 2 à 6 atomes de carbone et X représente un groupe méthylène pouvant être substitué par un ou deux groupes méthyle.

8. 1,5,6,7-tétrahydro-4H-indazol-4-one de la formule 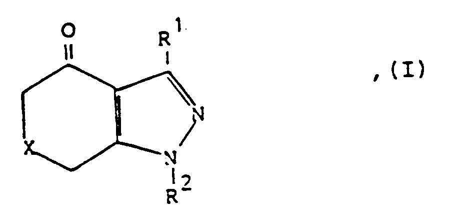 dans laquelle
R¹ est un radical phényle qui peut être éventuellement substitué par un atome halogène et R² un groupe alkyle linéaire ou ramifié avec 1 à 6 atomes de carbone qui peut encore être substitué par un radical phényle ou halogénophényle, et
X signifie un groupe méthylène qui peut être substitué par un ou deux groupes méthyle dans la mesure où X représente un groupe méthylène substitué par un ou deux groupes méthyle au cas où R¹ représente un radical phényle et R² un groupe méthyle.

9. Dérivé de pyrazole de la formule 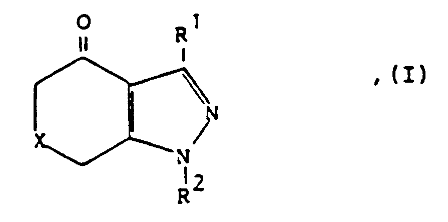 dans laquelle
R¹ et R² sont tels que définis dans la revendication 1 et X représente une liaison simple ou le groupe éthylène dans la mesure où X ne représente pas une liaison simple, lorsque R¹ représente un atome d'hydrogène et R² un groupe phényle.

10. Procédé pour la préparation des composés de la formule I selon les revendications 7, 8 et 9, caractérisé en ce que
a.) pour la préparation de la formule I dans laquelle R¹ possède les significations mentionnées dans les revendications 7 à 9 à l'exception d'un atome d'hydrogène, on met en réaction un composé de la formule II 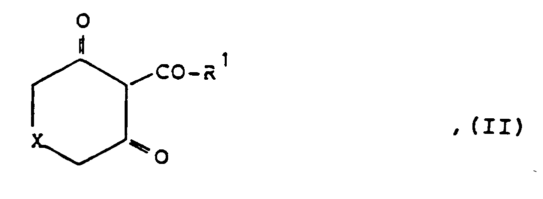 dans laquelle X est tel que défini dans les revendications 7 à 9 et R¹ possède les significations citées dans les revendications 7 à 9 à l'exception de l'atome d'hydrogène, avec une hydrazine de la formule III
H₂N - NH - R² ,(III)
dans laquelle R² est tel que défini dans les revendications 7 à 9 ou
b.) pour la préparation des composés de la formule I, dans laquelle R¹ signifie un atome d'hydrogène, on met en réaction un composé de la formule IV 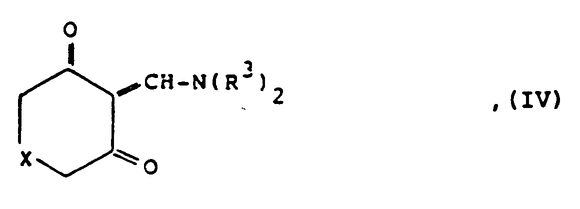 dans laquelle X est tel que défini dans les revendications 7 à 9 et R³ signifie un groupe alkyle avec une hydrazine de la formule III.
